# EUROPÄISCHE PATENTSCHRIFT

(11) **EP 1 242 349 B1**
(45) Veröffentlichungstag und Bekanntmachung des Hinweises auf die Patenterteilung: **20.02.2013**
(21) Anmeldenummer: 00991585.1
(22) Anmeldetag: 07.12.2000
(51) Int. Cl.: C07C 39/12, C07C 37/68

(54) **BIS(4-HYDROXYARYL)ALKANE**
BIS(4-HYDROXYARYL)ALKANES
BIS(4-HYDROXYARYL)ALCANES

(30) Priorität: 20.12.1999 DE 19961566
(43) Veröffentlichungstag der Anmeldung: 25.09.2002
(73) Patentinhaber: Bayer MaterialScience AG, 51368 Leverkusen (DE)
(72) Erfinder: NEUMANN, Rainer, 47803 Krefeld (DE); LANZE, Rolf, 47804 Krefeld (DE); HEYDENREICH, Frieder, 40593 Düsseldorf (DE); BÖDIGER, Michael, League City, TX 77573 (US); PREIN, Michael, B-2930 Brasschaat (BE)
(74) Vertreter: BIP Patents
(86) Internationale Anmeldenummer: PCT/EP2000/012324
(87) Internationale Veröffentlichungsnummer: WO 2001/046104

(56) Entgegenhaltungen:
- EP-A1- 0 343 349
- DE-A- 19 848 026
- US-A- 5 629 457

## Beschreibung

Die vorliegende Anmeldung betrifft ein Verfahren zur Herstellung von Bis(4-hydroxyaryl)alkanen hoher Reinheit aus Addukten von Bis(4-hydroxyaryl)alkanen und aromatischen Hydroxyverbindungen, die durch sauer katalysierte Umsetzung der aromatischen Hydroxyverbindungen mit Ketonen erhalten werden.

Bisphenole als Kondensationsprodukte von Phenolen und Carbonylverbindungen sind Ausgangsstoffe oder Zwischenprodukte zur Herstellung einer Vielzahl kommerzieller Produkte. Von besonderer technischer Bedeutung ist das Kondensationsprodukt aus der Reaktion zwischen Phenol und Aceton, 2,2-Bis(4-hydroxyphenyl)-propan (BPA). BPA dient als Ausgangstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Technisch relevante Herstellmethoden für BPA sind bekannt und beruhen auf der säurekatalysierten Umsetzung von Phenol mit Aceton, wobei ein bevorzugt Phenol-Aceton-Verhältnis von größer 5 : 1 in der Reaktion eingestellt wird. Als saure Katalysatoren können homogene wie auch heterogene Brönsted- oder Lewissäuren genutzt werden, so beispielsweise starke Mineralsäuren wie Salz- oder Schwefelsäure. Bevorzugt kommen gelförmige oder makroporöse sulfonierte vernetzte Polystyrolharze (saure Ionentauscher) zum Einsatz.

Bei der Umsetzung von Phenol mit Aceton in Gegenwart saurer Katalysatoren entsteht eine Produktmischung, die neben nicht umgesetzten Phenol und gegebenenfalls Aceton in erster Linie BPA und Wasser enthält. Daneben treten in geringen Mengen typische Nebenprodukte der Kondensationsreaktion auf, so beispielsweise 2-(4-hydroxyphenyl)-2-(2-hydroxyphenyl)propan (o,p-BPA), substituierte Indane, Hydroxyphenyl-indanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton beeinträchtigen die Eignung von BPA zur Herstellung von Polymeren und müssen durch geeignete Verfahren abgetrennt werden. Insbesondere zur Herstellung von Polycarbonat werden hohe Reinheitsanforderungen an den Rohstoff BPA gestellt.

Eine Aufarbeitungs- und Reinigungsmethode von BPA erfolgt durch Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts als Suspensionskristallisation. Die BPA/Phenol-Adduktkristalle werden anschließend durch eine geeignete Apparatur zur Fest-Flüssig-trennung wie Drehfilter oder Zentrifugen von der Flüssigphase abgetrennt und der weiteren Reinigung zugeführt.

So erhaltene Adduktkristalle weisen typischerweise ein Reinheit von > 99 % BPA bezogen auf die Nebenkomponenten bei einem Phenolanteil von ca. 40 % auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden.

Der bei der Fest-Flüssigtrennung anfallende Flüssigstrom (Mutterlauge) enthält Phenol, BPA, bei der Reaktion entstandenes Wasser, nicht umgesetztes Aceton und ist angereichert an den bei der BPA-Herstellung typischerweise anfallenden Nebenkomponenten. Dieser Mutterlaugenstrom wird in die Reaktionseinheit zurückgeführt. Um die katalytische Aktivität der sauren Ionentauscher aufrecht zu erhalten wird zuvor entstandenes Wasser durch Destillation entfernt, wobei auch gegebenenfalls noch vorhandenes Aceton aus der Mutterlauge entfernt wird. Der so erhaltene entwässerte Reaktionsstrom wird um Phenol und Aceton ergänzt und in die Reaktionseinheit zurückgeführt. Alternativ können auch vor Durchführung der Suspensionskristallisation des BPA-Phenol-Addukts Wasser und Aceton ganz oder teilweise destillativ entfernt werden. Bei den genannten Destillationsschritten kann auch eine Teilmenge des in der Reaktionslösung vorhandenen Phenols destillativ abgetrennt werden.

Bei einer derartigen Kreislauffahrweise tritt als Problem auf, dass Nebenprodukte der BPA-Herstellung im Kreislaufstrom angereichert werden und zur Desaktivierung des Katalysatorsystem führen. Um eine übermäßige Anreicherung von Nebenkomponenten im Kreislaufstrom zu vermeiden, wird eine Teilmenge des Kreislaufstroms - gegebenenfalls nach teilweiser oder vollständiger destillativer Rückgewinnung von Phenol - aus der Prozesskette als sogenanntes BPA-Harz ausgeschleust.

Außerdem hat es sich als vorteilhaft erwiesen einen Teil oder die Gesamtmenge des Kreislaufstroms nach der Fest-Flüssigtrennung und vor oder nach der Abtrennung von Wasser und Restaceton über eine mit saurem Ionentauscher befüllte Umlagerungseinheit zu führen. Diese Einheit wird im allgemeinen bei höheren Temperaturen betrieben als die Reaktionseinheit. In dieser Umlagerungseinheit werden unter den vorherrschenden Bedingungen einige der im Kreislaufstrom vorhandenen Nebenkomponenten der BPA-Herstellung zu BPA isomerisiert, so dass die Gesamtausbeute an BPA erhöht werden kann.

Die im Anschluss an die oben beschriebene Suspensionskristallisation der Reaktionslösung und Fest-Flüssig-Trennung erhaltenen BPA-Phenol-Adduktkristalle werden weitergehenden Reinigungsschritten zugeführt, wobei die Abtrennung von Phenol und gegebenenfalls die Verringerung der Konzentration an Nebenkomponenten erzielt wird.

So können die Adduktkristalle aus Phenol, organischen Lösungsmitteln, Wasser oder Mischungen der genannten Lösungsmitteln gemäß einer Suspensionskristallisation umkristallisiert werden. Hierbei kann durch die Wahl geeigneter Lösungsmittel auch das in den Adduktkristallen vorhandene Phenol ganz oder teilweise abgetrennt werden. Das gegebenenfalls nach der Umkristallisation im BPA verbleibende Phenol wird anschließend durch geeignete destillative, desorptive oder extraktive Methoden gänzlich abgetrennt.

Alternativ kann das Phenol auch durch Ausschmelzverfahren aus den Adduktkristallen entfernt werden. Das BPA unterliegt aber bei diesen Verfahren thermischen Belastungen was zu unerwünschten Spaltungen von BPA führt.

EP 343349 beschreibt ein Verfahren zur Herstellung von Bisphenol A, bei dem Phenol aus dem BPA/Phenol-Addukt in zwei Schritten abgetrennt wird. Zunächst wird der größte Teil von Phenol mittels Destillation, Extraktion, Wasserdampf-Strippung etc. entfernt. In einem zweiten Schritt erfolgt eine Wasserdampf-Strippung.

Aufgabe war daher ein optimiertes Verfahren zur Auftrennung von Addukten von Bis(4-hydroxyaryl)alkanen und aromatischen Hydroxyverbindungen mit dem die Bis(4-hydroxyaryl)alkane (Bis(4-hydroxyaryl)alkanen/Arylhydroxy-Addukte) in hoher Reinheit anfallen.

Es wurde nun gefunden, dass dies Aufgabe durch eine spezielle Anordnung und Verfahrensweise gelöst werden kann.

Gegenstand der Erfindung ist daher die Verwendung eines Desorbers mit Stickstoff und vorgeschalteter Destillationseinheit zur Abtrennung von Bis(4-hydroxyaryl)alkanen und aromatischen Hydroxyverbindungen von unter produktschonenden Bedingungen aufgeschmolzenen Bis(4-hydroxyaryl)alkanen/Arylhydroxy-Addukten.

In einer bevorzugten Ausführungsform wird in dem Desorber (1) 2,2-Bis(4-hydroxyphenyl)propan aus 2,2-Bis(4-hydroxyphenyl)propan/Phenol-Addukten abgetrennt.

In dem Desorber (1) wird Desorption durchgeführt. Dabei wird das Phenol abgetrennt und das BPA-Fertig-produkt als Sumpfprodukt gewonnen. Der Desorber (1) besteht vorzugsweise aus Röhrenbündelwärmetauschern. Diese Röhrenbündelwämetauscher vorzugsweise aus Röhrenbündelwärmetauschern. Diese Röhrenbündelwämetauscher sind insbesondere stehend angeordnet und in ihrem unteren Teil mit einer Anzahl von Düsen ausgestattet, über die ein inertes heißes Gas, insbesondere Stickstoff eingetragen wird. Das Gas wird vorzugsweise im Kreis geführt. Die Beheizung (6) der Wärmetauscher erfolgt durch Rohre und über den äußeren Mantel. Die Zwischenräume zwischen den Wärmeaustauscherrohren sind produktseitig mit Keramikkugeln (Steatit) gefüllt. Der Produktraum des Desorbers ist zum größten Teil mit Flüssigkeit gefüllt.

Das abdestillierte Phenol gelangt mit dem Gas zu Kondensatoren (2) und wird dort der Phenolsammelvorlage (3) zugeführt.

Das aus den Kondensatoren (2) und Abscheidern (3) austretende Gas wird vorzugsweise in einem Waschturm (4) und gegebenenfalls Filter (5) gereinigt und über Kompressoren in den Desorber zurückgepumpt.

Zur Aufrechterhaltung eines niedrigen Sauerstoffgehalts im Kreislaufstrom wird vorzugsweise laufend Frischgas zugeführt.

Gegenstand der Erfindung ist auch ein Verfahren zur Abtrennung von Bis(4-hydroxyaryl)alkanen aus Addukten von Bis(4-hydroxyaryl)alkanen mit aromatischen Hydroxyverbindungen, das dadurch gekennzeichnet ist, dass die im Prozess anfallenden kristallisierten, abfiltrierten und gereinigten Bis(4-hydroxyaryl)alkan/Arylhydroxy-Addukte unter produktschonenden Bedingungen aufgeschmolzen und durch Desorption mit Stickstoff und vorgeschalteter Destillation von den aromatischen Hydroxyverbindungen getrennt werden.

Die Trennung wird vorzugsweise derart durchgeführt, dass der Werte der aromatischen Hydroxyverbindungen unter 50 ppm liegt.

In einer bevorzugten Ausführungsform wird 2,2-Bis(4-hydroxyphenyl)propan aus 2,2-Bis(4-hydroxyphenyl)propan/Phenol-Adukkten abgetrennt.

Die Abtrennung wird durch Desorption mit gegebenenfalls vorgeschalteter Destillation durchgeführt.
Vorzugsweise wird dem Desorber eine Mischkristallschmelze mit einem Phenolanteil von 40 bis 5 %, bevorzugt 20 bis10 %, besonders bevorzugt 15 bis10 % zugeführt .
Die Temperatur der Schmelze im Desorber liegt im allgemeinen bei 160°C bis 210°C vorzugsweise 170°C bis 200°C ganz besonders bei 180°C bis 195°C. Die Temperatur wird vorzugsweise durch die zulaufende Schmelze und eine externe Beheizung eingestellt. Die Heizoberflächen der externen Beheizung liegt vorzugsweise bei165°C bis 230°C, insbesondere 185 bis 200°C.

Die in den Desorber eingeleitete Gasmenge beträgt vorzugsweise 100 bis 300 m³, insbesondere 150 - 250 m³ pro m³ BPA/Phenol-Adduktmischung.

Das Gas wird vorzugsweise vor Eintritt in den Desorber durch Vorheizen auf Temperaturen über 160°C bis 230°C, insbesondere auf Temperaturen von 185 bis 200°C eingestellt.

Als Gas wird Stickstoff, vorzugsweise sauerstofffreies Gas, eingeleitet. Das Gas wird vorzugsweise nach der Desorbtion durch Kühlung und Abtrennung des Phenols im Kreis gefahren. Dazu wird das Gas vorzugsweise gewaschen und durch Filtration gereinigt. Eine Menge des Gases ca. 1 - 5 % wird vorzugsweise aus dem Kreislaufstrom ausgespeist.

Das Gas im Kreislaufstrom sollte vorzugsweise einen Sauerstoffgehalt unter 10ppm, bevorzugt 3ppm, besonders bevorzugt 1 ppm haben.

Vorzugsweise wird der Kreislaufgasstrom von metallischem und keramischem Feststoffabrieb in gesinterten Filterkerzen befreit.

Die Kühlung des Gases (inkl. gegebenenfalls die Phenoleindüsung) wird so vorgenommen, dass in dem abgekühlten Gasstrom gegebenenfalls mitverdampfendes Produkt die Kühlerfläche des Kondensators und die stromabwärts installierten Apparate, Rohrleitungen und messtechnischen Einrichtungen nicht belegen.

Der Schmelzzufluss zum und vom Desorber wird vorzugsweise filtriert.

Der Stand im Desorber wird vorzugsweise 10-15 cm über dem Zulauf und der Packung eingestellt.

Das Gas vorzugsweise über Verteilerrohre und nach unten geöffnete Düsen durch einen mit Füllkörpern gefüllten Desorber im Gegenstrom zu der von oben herunterlaufenden Schmelze geführt wird.

Die Zahl der Düsen in den Verteilerrohren beträgt pro m² Querschnittsfläche vorzugsweise 800 bis1500.

Am Kopf des Desorbers wird vorzugsweise ein Überdruck von 15 bis 25 mbar eingestellt.

Die Verweilzeit der Produktschmelze im Desorber beträgt vorzugsweise weniger als 30 min.

Weiterer Gegenstand der Erfindung ist eine Vorrichtung zur Abtrennung von Bis(4-hydroxyaryl)alkanen und aromatischen Hydroxyverbindungen von Bis(4-hydroxyaryl)alkanen/Arylhydroxy-Addukten gekennzeichnet durch einen Desorber, der Röhrenbündelwärmetauscher enthält und wobei die Zwischenräume zwischen den Wärmeaustauscherrohren produktseitig mit Keramikkugeln (Steatit) gefüllt sind und der Produktraum zum größten Teil mit Flüssigkeit gefüllt ist und wobei die Wärmetauscher stehend angeordnet sind und in ihrem unteren Teil mit einer Anzahl von Düsen ausgestattet sind, über die ein inertes heißes Gas, insbesondere Stickstoff eintragbar ist, mit einer Ableitung für das abdestillierte Phenol mit dem Gas zu einem Kondensator und einer daran angeschlossenen Phenolsammelvorlage sowie einem an die Phenolsammelvorlage angeschlossenen Waschturm und gegebenenfalls Filter zur Reinigung des aus den Kondensatoren und Abscheidern austretenden Gases und einer Gasrückführung in den Desorber.

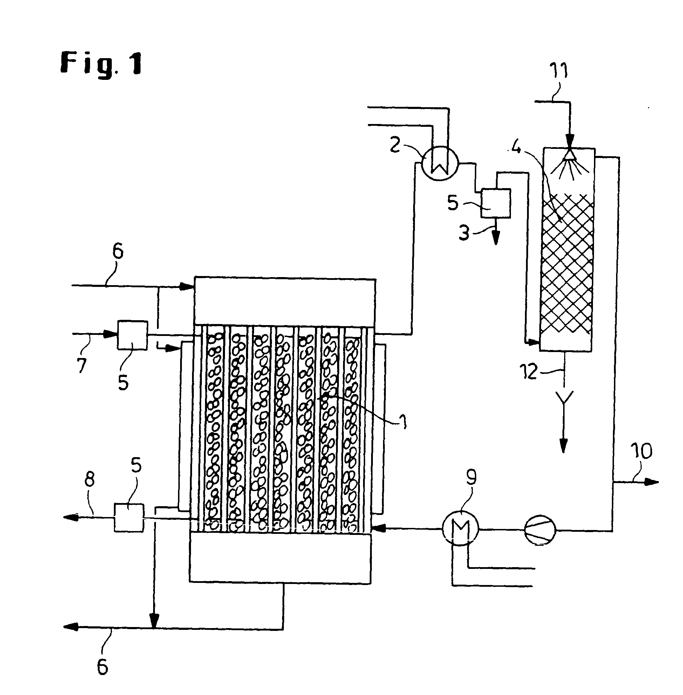

In Fig 1 bedeutet:
- (1): Desorber
- (2): Kühler
- (3): Phenolablauf
- (4): Gas-Waschturm
- (5): Filter
- (6): Beheizung
- (7): Mischkristalleinleiteeinheit
- (8): Produktentnahmeeinheit
- (9): Erhitzer
- (10): Gas-Ausspeisung
- (11): Waschwassereinleitung
- (12): Waschwasserablauf

Die nachfolgenden Beispiele dienen zur Erläuterung der Erfindung. Die Erfindung ist nicht auf die Beispiele limitiert. Wenn nichts anderes angegeben stehen Prozentangaben für Gewichtsprozente.

### Beispiel

### Beispiel 1

Die bei der säurekatalysierlen Umsetzung von Phenol und Aceton mit anschließender Suspensionskristallisation anfallenden BPA/Phenol-Adduktkristalle werden durch ein Drehfilter von der Flüssigphase abgetrennt und gereinigt und zur Trennung von BPA vom Phenol einer Abtrennungseinheit zugeführt. Hierzu wird eine Mischkristallschmelze mit einem Phenolanteil von 15 % in einen Desorber (1) gepumpt. Die Temperatur der Schmelze im Desorber wird durch Schmelzzulauf und Beheizung auf 190°C eingestellt, wobei die Temperatur der Heizoberflächen 200°C beträgt.

Zur Desorption wird Stickstoff (N₂) eingesetzt, wobei die Stickstoffinenge 200 m³/m³ BPA/Phenol-mischung beträgt und die die N₂-Temperatur vor Eintritt in den Desorber durch Vorheizen auf 195°C eingestellt wird. Der Stickstoff wird nach Desorption durch Kühlung und Abtrennung des Phenols im Kreis gefahren, gewaschen und von BPA- bzw. BPA-Phenolsublimat durch Filtration gereinigt. Im Verhältnis zum Kreislaufstrom wird eine Menge von 3 % an N₂ ausgespeist.

Der Stickstoff wird dabei über Verteilerrohre und nach unten geöffnete Düsen durch einen mit Füllkörpern gefüllten Desorber im Gegenstrom zu der von oben herunterlaufenden Schmelze geführt, wobei die Zahl der Düsen pro m² Querschnittsfläche etwa 1100 beträgt. Am Kopf des Desorbers wird ein Überdruck von etwa 20 mbar eingestellt, der Stand im Desorber wird bei 15 cm über dem Zulauf und der Packung eingestellt.

Zur Erzielung einer hohen BPA-Qualität ist darauf zu achten, dass der Sauerstoffgehalt im Kreislaufstickstoff unter 1 ppm liegt und der Kreislauftstickstoff von metallischem und keramischem Feststoffabrieb in gesinterten Filterkerzen befreit wird sowie der Schmelzzufluss zum und vom Desorber filtriert wird. Darüber hinaus sollte die Verweilzeit der Produktschmelze in der Desorbereinheit < 30 min betragen.

Hierdurch wird ein BPA mit hoher Reinheit (>99.5%) und geringen Phenolgehalten (<50ppm) erhalten.

## Patentansprüche

1. Verwendung eines Desorbers (1) mit Stickstoff und vorgeschalteter Destillationseinheit zur Abtrennung von Bis(4-hydroxyaryl)alkanen und aromatischen Hydroxyverbindungen von unter produktschonenden Bedingungen aufgeschmolzenen Bis(4-hydroxyaryl)alkanen/Arylhydroxy-Addukten.

2. Verfahren zur Abtrennung von Bis(4-hydroxyaryl)alkanen aus Addukten von Bis(4-hydroxyaryl)alkanen mit aromatischen Hydroxyverbindungen, das **dadurch gekennzeichnet ist, dass** die im Prozess anfallenden kristallisierten, abfiltrierten und gereinigten Bis(4-hydroxyaryl)alkan/Arylhydroxy-Addukte unter produktschonenden Bedingungen aufgeschmolzen und durch Desorption mit Stickstoff und vorgeschalteter Destillation von den aromatischen Hydroxyverbindungen getrennt werden.

3. Verfahren gemäß Anspruch 2, **dadurch gekennzeichnet, dass** 2,2-Bis(4-hydroxyphenyl)propan von 2,2-Bis(4-hydroxyphenyl)propan/Phenol-Addukten abgetrennt wird.

4. Verfahren gemäß Anspruch 2, **dadurch gekennzeichnet, dass** die in den Desorber eingeleitete Gasmenge 100 bis 300 m³ pro m³ BPA/Phenol-Adduktmischung und die Gastemperatur über 160°C bis 230°C beträgt.

5. Verfahren gemäß Anspruch 2, **dadurch gekennzeichnet, dass** das Gas nach der Desorption durch Kühlung und Abtrennung des Phenols im Kreis gefahren wird und eine ca. 1 - 5 % des Gases aus dem Kreislaufstrom ausgespeist wird.

6. Vorrichtung zur Abtrennung von Bis(4-hydroxyaryl)alkanen und aromatischen Hydroxyverbindungen von Bis(4-hydroxyaryl)alkanen/Arylhydroxy-Addukten **gekennzeichnet durch** einen Desorber (1), der Röhrenbündelwärmetauscher enthält und wobei die Zwischenräume zwischen den Wärmeaustauscherrohren produktseitig mit Keramikkugeln (Steatit) gefüllt sind und der Produktraum zum größten Teil mit Flüssigkeit gefüllt ist und wobei die Wärmetauscher stehend angeordnet sind und in ihrem unteren Teil mit einer Anzahl von Düsen ausgestattet sind, über die ein inertes heißes Gas, insbesondere Stickstoff eintragbar ist, mit einer Ableitung für das abdestillierte Phenol mit dem Gas zu einem Kondensator (2) und einer daran angeschlossenen Phenolsammelvorlage (3) sowie einem an die Phenolsammelvorlage angeschlossenen Waschturm (4) und gegebenenfalls Filter (5) zur Reinigung des aus den Kondensatoren (2) und Abscheidern (3) austretenden Gases und einer Gasrückführung in den Desorber (1).

## Claims

1. Use of a desorber (1) with nitrogen and an upstream distillation unit for separation of bis(4-hydroxyaryl)alkanes and aromatic hydroxyl compounds from bis(4-hydroxyaryl)alkanes/aryl-hydroxyl adducts which have been melted under non-product-destructive conditions.

2. Process for separating bis(4-hydroxyaryl)alkanes from adducts of bis(4-hydroxyaryl)alkanes with aromatic hydroxyl compounds, **characterized in that** the bis(4-hydroxyaryl)alkane/arylhydroxyl adducts which have crystallized and been filtered off and purified and have been obtained in the process are melted under non-product-destructive conditions and separated from the aromatic hydroxyl compounds by desorption with nitrogen and upstream distillation.

3. Process according to Claim 2, **characterized in that** 2,2-bis(4-hydroxyphenyl)propane is removed from 2,2-bis(4-hydroxyphenyl)propane/phenol adducts.

4. Process according to Claim 2, **characterized in that** the amount of gas introduced into the desorber is 100 to 300 m³ per m³ of BPA/phenol adduct mixture and the gas temperature is more than 160°C to 230°C.

5. Process according to Claim 2, **characterized in that** the gas after desorption is circulated by cooling and removing the phenol, and approx. 1-5% of the gas is fed out of the circulation stream.

6. Apparatus for separation of bis(4-hydroxyaryl)alkanes and aromatic hydroxyl compounds from bis(4-hydroxyaryl)alkanes/arylhydroxyl adducts, **characterized by** a desorber (1) which comprises shell-and-tube heat exchangers, and wherein the interstices between the heat exchanger tubes are filled on the product side with ceramic spheres (steatite) and the product space is for the most part filled by liquid, and wherein the heat exchangers are arranged vertically and are equipped in the lower portion thereof with a number of nozzles through which an inert hot gas, especially nitrogen, can be introduced, with an outlet for the phenol distilled off with the gas to a condenser (2) and a phenol receiver (3) connected thereto, and a wash tower (4) connected to the phenol receiver and optionally filters (5) for purification of the gas leaving the condensers (2) and separators (3), and a gas recycle line into the desorber (1).

## Revendications

1. Utilisation d'un désorbeur (1) avec de l'azote et une unité de distillation raccordée en amont, pour la séparation de bis(4-hydroxyaryl)alcanes et de composés hydroxylés aromatiques d'avec des adduits bis(4-hydroxyaryl)alcanes/arylhydroxy fondus dans des conditions ménageant le produit.

2. Procédé pour la séparation de bis(4-hydroxy-aryl)alcanes à partir d'adduits de bis(4-hydroxyaryl)-alcanes avec des composés hydroxylés aromatiques, **caractérisé en ce qu'**on fait fondre dans des conditions ménageant le produit les adduits bis(4-hydroxyaryl)alcane/arylhydroxy cristallisés apparaissant dans le processus, séparés par filtration et purifiés, et on les sépare des composés hydroxylés aromatiques par désorption avec de l'azote et distillation raccordée en amont.

3. Procédé selon la revendication 2, **caractérisé en ce qu'**on sépare du 2,2-bis(4-hydroxyphényl)propane d'avec des adduits 2,2-bis(4-hydroxyphényl)propane/phénol.

4. Procédé selon la revendication 2, **caractérisé en ce que** la quantité de gaz introduite dans le désorbeur vaut de 100 à 300 m³ par m³ de mélange d'adduit BPA/phénol et la température du gaz est de plus de 160 °C à 230 °C.

5. Procédé selon la revendication 2, **caractérisé en ce qu'**après la désorption on met le gaz en circuit par refroidissement et séparation du phénol et on évacue du courant en circuit environ 1 - 5 % du gaz.

6. Dispositif pour la séparation de bis (4-hydroxyaryl)alcanes et de composés hydroxylés aromatiques d'avec des adduits bis(4-hydroxyaryl)-alcanes/arylhydroxy au moyen d'un désorbeur (1) qui contient des échangeurs thermiques à faisceaux de tubes et les espaces intermédiaires entre les tubes échangeurs thermiques étant garnis du côté du produit de sphères céramiques (stéatite) et l'espace contenant le produit étant en majeure partie rempli de liquide, et les échangeurs thermiques étant disposés verticalement et munis dans leur partie inférieure d'un certain nombre de buses, par lesquelles peut être introduit un gaz inerte chaud, en particulier de l'azote, avec une dérivation pour le phénol séparé par distillation avec le gaz, conduisant à un condenseur (2) et à un collecteur de phénol (3) raccordé à ce dernier ainsi qu'à une tour de lavage (4) raccordée au collecteur de phénol et éventuellement des filtres (5) destinés à la purification du gaz sortant des condenseurs (2) et séparateurs (3) et à un recyclage du gaz dans le désorbeur (1).
